# EUROPÄISCHE PATENTSCHRIFT

(11) **EP 3 003 198 B1**
(45) Veröffentlichungstag und Bekanntmachung des Hinweises auf die Patenterteilung: **12.07.2017**
(21) Anmeldenummer: 14723830.7
(22) Anmeldetag: 15.05.2014
(51) Int. Cl.: A61B 90/50, A61G 13/10

(54) **ADAPTIERVORRICHTUNG FÜR EINEN OP-TISCH**
ADAPTER DEVICE FOR AN OPERATING TABLE
DISPOSITIF ADAPTATEUR POUR TABLE D'OPÉRATION

(30) Priorität: 24.05.2013 DE 102013105374
(43) Veröffentlichungstag der Anmeldung: 13.04.2016
(73) Patentinhaber: Aesculap AG, 78532 Tuttlingen (DE)
(72) Erfinder: VOGTHERR, Robert, 78532 Tuttlingen (DE); BECK, Thomas, 78591 Durchhausen (DE); MORALES, Pedro, 78532 Tuttlingen (DE)
(74) Vertreter: Winter, Brandl, Fürniss, Hübner, Röss, Kaiser, Polte - Partnerschaft mbB
(86) Internationale Anmeldenummer: PCT/EP2014/059934
(87) Internationale Veröffentlichungsnummer: WO 2014/187721

(56) Entgegenhaltungen:
- WO-A1-2013/060581
- DE-A1-102009 021 222
- US-A- 4 796 846
- US-A- 6 023 800
- US-A1- 2013 092 058
- US-A1- 2013 123 911

## Beschreibung

Die vorliegende Erfindung betrifft eine Adaptiervorrichtung zur lösbaren Befestigung von Halteinstrumenten, Zusatzinstrumenten, Instrumentenzureichungstischen und dergleichen an einer Seitenschiene eines Operationstischs.

Bei der folgenden Beschreibung werden Halteinstrumente, Zusatzinstrumente, Instrumentenzureichungstische und dergleichen kurz als Zusatzeinrichtungen bezeichnet.

Bei chirurgischen Eingriffen an einem Patienten auf einem Operationstisch werden Zusatzeinrichtungen üblicherweise an genormten Seitenschienen mit rechteckigem Querschnitt, die sich seitlich am Operationstisch befinden, angeordnet und befestigt. Moderne Operationstische sind in mehrere Tischsegmente unterteilt, die relativ zueinander verstell- und positionierbar sind, damit der Operationstisch an die Art des Eingriffs, die jeweilige Operationssituation sowie Größe und Gestalt des Patienten angepasst werden kann. Herzchirurgische Eingriffe beispielsweise erfordern oftmals, dass der Oberkörper des Patienten in einem definierten Winkel höher als Beine und Hüfte gelagert wird. Dies wird erreicht, indem kopfseitige Segmente des Operationstischs schräg gestellt werden.

Die Seitenschienen des Operationstisches sind wie der Tisch selbst entsprechend segmentiert. In der Regel ist jedem Tischsegment ein Seitenschienensegment zugeordnet, an dem Zusatzeinrichtungen befestigt werden können. Verstellungen eines Tischsegments bewirken entsprechende Positionsänderungen der jeweiligen Seitenschienensegmente und damit auch den dort angeordneten Zusatzein-richtungen. Unter bestimmten Umständen kann dann von Nachteil sein, dass sich diese nicht mehr in der vorherigen, sorgsam ausgerichteten Lage und Position befinden.

Aus dem Stand der Technik sind Vorrichtungen bekannt, mit denen man zuvor genannte Zusatzeinrichtungen an einem Operationstisch anordnen und befestigen kann. Zusatzschienen, die an einer Seitenschiene des Operationstisches befestigt werden und neben der Letzteren zusätzliche Möglichkeiten zur Befestigung von Zubehör bieten, sind starr mit der Tischschiene verbunden und folgen daher jeder Lageänderung. Ferner sind Spannkloben bekannt, die man an einer Tischschiene festgeklemmen kann und die über ein Kugelgelenk verfügen. Solche Vorrichtungen werden z.B. als Infusionshalter oder für einen Instrumentenzureichungstisch genutzt. Dabei ist über das Kugelgelenk eine vertikale Ausrichtung des Halters bzw. eine horizontale Ausrichtung des Instrumentenzureichungstischs möglich.

Die Druckschrift WO 2013/060581 offenbart eine Adaptationsvorrichtung zur lösbaren Verbindung von Zusatzinstrumenten an einer Seitenschiene eines Operationstischs, gemäß dem Oberbegriff des Anspruchs 1. Hierfür weist die aus der WO 2013/060581 bekannte Adaptationsvorrichtung eine Klemmvorrichtung, mittels der die Adaptationsvorrichtung an der Seitenschiene eines Operationstisches klemmbar fixierbar ist, und ein Längsprofil auf, an dem Zusatzinstrumente lösbar befestigbar sind. Diese bekannte Vorrichtung weist im Gegensatz zur vorliegenden Erfindung kein Gelenk auf, über das das Längsprofil bzw. die Simulationsschiene schwenkbar an der Klemmvorrichtung fixierbar ist.

Die Druckschrift US 4 796 846 offenbart beispielsweise eine Klemmvorrichtung, die einen Schaft eines chirurgischen Instruments direkt aufnehmen kann. Die gesamte Klemmvorrichtung kann gedreht bzw. verschwenkt werden, wenn der Operationstisch verstellt wird. Allerdings muss bei der Verwendung einer solchen Klemmvorrichtung die Position einer jeden Klemmvorrichtung eines jeden chirurgischen Instruments einzeln justiert werden, wenn der OP-Tisch verstellt wird.

Die Druckschrift US 2013/0092058 A1 offenbart eine Halterungsvorrichtung, mit der ein medizinisches Tablett relativ zu der Oberfläche eines OP-Tischs verschwenkbar über diesem OP-Tisch gehalten werden kann. Die Halterungsvorrichtung hat eine Klemmvorrichtung, die eine Seitenschiene eines OP-Tischs aufnehmen kann. In die Klemmvorrichtung ist ein Trägerteil eingesetzt, welches an seinem dem OP-Tisch abgewandten Ende abgerundet ist. Auf diesem abgerundeten Ende des Trägerteils liegt das medizinische Tablett verschwenkbar auf.

Die Druckschrift US 2013/0123911 A1 beschreibt ein kardiologisches Instrument zum Operieren der Herzklappen. Dieses Instrument ist über ein Gelenk mit einer Klemmvorrichtung, welche auf eine Seitenschiene eines OP-Tischs klemmbar ist, verschwenkbar verbunden. Das Gelenk befindet sich hierbei direkt zwischen dem Instrument und der dazugehörigen Klemmvorrichtung.

Weiterhin offenbart die Druckschrift US 6 023 800 eine an einen OP-Tisch klemmbare Leiste und die Druckschrift DE 10 2009 021 222 offenbart eine Halteeinrichtung zur Befestigung von Gegenständen an einer Halteschiene eines Operationstisches mit zwei Klemmbacken, welche in der Klemmstellung durch einen elastischen Kraftspeicher dauerhaft klemmend an der Halteschiene anliegen.

Aus der DE 403952 ist eine Kupplungsvorrichtung zur Verbindung eines Instrumententischs mit einem Operationstisch oder dergleichen bekannt, bei der ein Instrumententisch mit einer Klemme an einer Längsschiene des Operationstischs verschiebbar befestigt ist. Die Klemme trägt eine höhenverstellbare Stützstange mit einem Kardangelenk. In dem Kardangelenk ist eine Stange drehbar, die an einer Seite den Instrumententisch und an der gegenüberliegenden Seite ein Gegengewicht trägt. Das Gegengewicht hält den Instrumententisch in jeder Stellung des Operationstischs in der Waage.

Die Vorrichtungen nach dem Stand der Technik sind in nachteiliger Weise entweder Spannkloben mit Möglichkeiten zu verstellen, die aber jeweils nur eine Zusatzeinrichtung aufnehmen können, oder es sind Zusatzschienen, die zwar mehrere Zusatzeinrichtungen tragen können, aber relativ zum jeweiligen Operationstischsegment starr angeordnet sind und keine Verstell- oder Einstellmöglichkeit bieten. Bei einstellbarer Anordnung mehrerer Zusatzeinrichtungen mittels Spannkloben muss in nachteiliger Weise jede Zusatzeinrichtung bei Verstellung des Operationstisches oder einzelner seiner Segmente einzeln auf die neue Konfiguration des Tisches eingestellt werden.

Das ist zeitaufwändig und unpraktisch, besonders wenn die Verstellungen des Operationstischs während einer Operation notwendig sind.

Schließlich wurden OP-Tische in der Regel mit sterilen Tüchern abgedeckt, sodass dadurch auch die Tischschienen unter den Tüchern verschwinden. Dadurch wird ein exaktes Positionieren und Befestigen einer Zusatzeinrichtung an der verdeckten Tischschiene erschwert.

Ausgehend von dem zuvor beschriebenen Stand der Technik liegt der Erfindung die Aufgabe zugrunde, eine Adaptiervorrichtung zur lösbaren Befestigung von Zusatzeinrichtungen an einer der Seitenschienen eines Operationstisches zu schaffen, die eine einfachere Handhabung ermöglicht. Vorzugsweise soll eine Adaptiervorrichtung bereitgestellt werden, die universell für übliche Zusatzeinrichtungen verwendbar ist, eine Mehrzahl von Zusatzeinrichtungen aufnehmen und an einem Operationstisch befestigen kann, durch einen Benutzer einfach, sicher, genau sowie schnell verstell- und einstellbar ist sowie ausgerichtet werden kann und bei Positions-änderungen am Operationstisch oder bestimmter seiner Segmente eine rasche Ausrichtung der dort angeordneten Zusatzeinrichtungen ermöglicht.

Diese Aufgabe wird durch eine Adaptiervorrichtung mit den Merkmalen des Anspruchs 1 gelöst.

Die Erfindung vereint die Vorteile einer adaptierbaren Zusatzschiene mit denen eines üblichen Spannklobens und ermöglicht bei Verwendung mit einem Operationstisch mit segmentierten Seitenschienen einen einfachen Ausgleich der Winkelverstellungen von Segmenten eines Operationstisches. In der einfachsten Ausgestaltung hat das Gelenk einen einzigen Schwenk-Freiheitsgrad mit einer Verschwenkmöglichkeit des Längsprofils in einer vertikalen Ebene. Das Längsprofil ermöglicht es, darauf eine Mehrzahl von Zusatzeinrichtungen anzuordnen und ist mittels der Klemmvorrichtung einfach an einer genormten Seitenschiene des Operationstischs anzuordnen. Durch das zwischen Klemmvorrichtung und Längsprofil ausgebildete Gelenk ist das Längsprofil in vorteilhafter Weise zusammen mit sämtlichen darauf angeordneten Zusatzeinrichtungen verstell- und justierbar. Wird zum Beispiel ein Segment eines Operationstischs verstellt, können alle an diesem Segment angeordneten Zusatzeinrichtungen durch einen einzigen und einfachen Bedienvorgang einheitlich, gleichzeitig, rasch und einfach analog der neuen Stellung des Segments angepasst ausgerichtet werden. Die Zusatzeinrichtungen sind auf dem Längsprofil vorzugsweise in dessen Längsrichtung verschiebbar aufgenommen, so dass sie einfach relativ zueinander positioniert werden können. Auf dem Längsprofil können neben Zusatzeinrichtungen auch weitere Adapter angebracht werden. Dadurch wird hohe Flexibilität der erfindungsgemäßen Adaptiervorrichtung ermöglicht. In anderen Worten ausgedrückt hat die erfindungsgemäße Adaptiervorrichtung ein Montageteil beispielsweise in Form der genannten Klemmvorrichtung oder Schraubzwinge, an die ein OP-Tischschienen-Simulationsteil über ein arretierbares Scharnier angelenkt ist. Das OP-Tischschienen-Schimulationsteil hat folglich das die OP-Tischschiene nachbildende (simulierende) Längsprofil, das sich im montierten Zustand oberhalb eines sterilen Tuchs im Sterilraum angeordnet und dafür gut sichtbar ist. Ferner befindet sich das arretierbare Scharnier auf einer Seite der Klemmvorrichtung, die dem Klemmeingriffsmitteln abgewandt ist und damit ebenfalls oberhalb des sterilen Tuchs im Sterilraum liegt. Somit kann das Längsprofil während einer OP in Form einer Nickbewegung bezogen auf die Längsprofilerstreckung geschwenkt werden.

Das Gelenk kann auf verschiedenste Art und Weise arretierbar ausgestaltet werden. Insbesondere kann das Gelenk mittels Formschluss arretierbar sein und arretiert werden, vorzugsweise indem ein Rastelement in eine Rastausnehmung oder in eine von mehreren Rastausnehmungen des Gelenks eingreift. Eine formschlüssige Arretiermöglichkeit ist mit Vorteil schnell, einfach und sicher zu bedienen. Das Rastelement kann ein federbelasteter Zugbolzen sein, der in Ruhelage, in welche er vorgespannt ist, in eine der Rastausnehmungen eingreift und durch manuell aufgebrachten Zug gegen die Federbelastung aus der Rastausnehmung gelöst werden kann.

Alternativ kann das Gelenk durch Kraftschluss arretierbar oder arretiert sein, vorzugsweise, indem eine Spannschraube das Gelenk klemmt. Über Kraftschluss zu arretieren, bietet den Vorteil sehr feiner individuell auswählbarer Einstellmöglichkeiten.

Das Gelenk der erfindungsgemäßen Adaptiervorrichtung kann auch mit mehreren Bewegungsfreiheitsgraden ausgestattet sein, insbesondere als Dreh- oder Kugelgelenk ausgebildet sein. Die Auswahl geschieht je nach Anzahl der gewünschten Freiheitsgrade für Einstellungen der Adaptiervorrichtung.

Nach einer Ausgestaltungsform der Erfindung bildet die Klemmvorrichtung einen ersten Teil des Gelenks aus, während das Längsprofil oder eine mit dem Längsprofil verbundene Einheit den mit dem ersten Gelenkteil zusammenwirkenden Gegenpart ausbildet. Auf diese Weise ist die Adaptiervorrichtung sehr kompakt und stabil und kann nahezu ohne Einschränkungen der Zugänglichkeit des Operationstischs an diesem angeordnet werden.

Die Klemmvorrichtung kann insbesondere nach Art eines Spannklobens ausgebildet oder ein Spannkloben sein. Ein Spannkloben kann vorteilhaft an einer Seitenschiene eines Operationstischs angeordnet werden. Er weist in der Regel eine Vertiefung oder Nut auf, in die eine übliche Seitenschiene leicht eingeführt werden kann. Die Vertiefung oder Nut kann mit einer Hinterschneidung oder mit einer Seitenschiene übergreifenden Vorsprüngen ausgebildet sein (z. B. Schwalbenschwanz-Querschnitt), so dass die Seitenschiene sicher und in korrekter Position aufgenommen werden kann. Die Seitenschiene ist in der Vertiefung oder Nut mittels einer Klemmvorrichtung, z.B. in Form einer Spannschraube oder Spannhebels oder einer dadurch betätigten Druckplatte zu klemmen. Mit einer Druckplatte wird die auf die Seitenschiene einwirkende Flächenpressung verringert, so dass Bildung von Druckstellen weitgehend vermieden werden können.

Insbesondere dann, wenn die Klemmvorrichtung einen ersten Teil des Gelenks ausbildet und somit sich die Schwenkachse im Wesentlichen in Höhe der Seitenschiene des Operationstisches befindet, ist es von besonderem Vorteil, wenn die Adaptiervorrichtung über einen Arm oder Ausleger verfügt, der dann das Längsprofil mit Abstand (Höhenabstand) von der Schwenkachse des Gelenkes trägt. Der Arm kann dabei außerdem dazu herangezogen werden, die Höhe einer OP-Tisch-Auflage auszugleichen. Durch geeignete Ausgestaltung des Arms bzw. der Verbindung zwischen Arm und Längsprofil ist es außerdem mit einfachen Maßnahmen möglich, Einfluss auf den seitlichen Abstand zwischen OP-Tisch unf Längsprofil zu nehmen..

Von besonderem Vorteil ist es, wenn der Arm als Handgriff bei einer Justierung oder Verstellung der Adaptiervorrichtung dient. Er kann ferner insbesondere eine Aussparung oder einen Freiraum aufweisen, die als Handgriff nutzbar ist. Mit besonderem Vorteil kann der Arm winkelförmig mit zwei Armelementen ausgebildet sein. Dabei kann jedes Armelement einerseits an dem Gelenk und andererseits an dem Längsprofil angeordnet sein. In dieser Ausführungsform ist das Längsprofil besonders stabil durch den Arm gesichert und getragen und der Arm ist als Handhabe ausgebildet einfach geformt, wodurch einfache Reinigung möglich ist. Um Bedienung angenehm zu machen und ein Verletzungsrisiko zu minimieren, können die Kanten der Adaptiervorrichtung, insbesondere die Kanten des als Handhabe ausgebildeten Arms, abgerundet sein.

Nach einer weiteren Ausführungsform kann der Querschnitt des Längsprofils dem Querschnitt einer genormten Seitenschiene eines Operationstisches entsprechen. Dadurch ist sichergestellt, dass übliche Zusatzeinrichtungen für Seitenschienen auch gemeinsam mit der erfindungsgemäßen Adaptiervorrichtung verwendet werden können. Alternativ kann der Querschnitt des Längsprofils rund sein. In beiden Fällen kann das Längsprofil Endanschläge aufweisen, die versehentliches Lösen der darauf angeordneten Zusatzeinrichtungen, beispielsweise bei Ausrichten in Längsrichtung des Längsprofils, verhindern.

Für Längsprofile mit rundem Querschnitt ist von Vorteil, wenn diese bereichsweise, insbesondere endseitig, abgeflacht oder mit wenigstens einer Ausnehmung ausgebildet sind, um Anordnen und Lösen von Zusatzeinrichtungen darauf zu erleichtern. Die Zusatzeinrichtungen sind dann nach dem Schlüssel-Schloss-Prinzip im Bereich der Abflachung oder Ausnehmung mit dem Längsprofil kombinierbar, d.h. auf diesem anzubringen oder zu entfernen. Außerhalb eines abgeflachten Bereiches ist Anbringen oder Entfernen von Zusatzeinrichtungen nicht möglich, was die Sicherheit in der Handhabung erhöht.

Weitere Merkmale und Vorteile der vorliegenden Erfindung ergeben sich aus der folgenden beispielhaften Beschreibung einer besonders bevorzugten Ausführungsform der Erfindung anhand der Figuren. Dabei zeigt:
- **Fig. 1**: eine erste Ausführungsform einer Adaptiervorrichtung nach der Erfindung in perspektivischer Ansicht,
- **Fig. 2**: die Adaptiervorrichtung der Fig. 1 in Seitenansicht,
- **Fig. 3**: die Adaptiervorrichtung der Fign. 1 und 2 in einer Aufsicht in einer gegenüber den Fign. 1 und 2 geschwenkten Position und
- **Fig. 4**: eine andere Ausführungsform einer Adaptiervorrichtung gemäß der Erfindung in perspektivischer Ansicht.

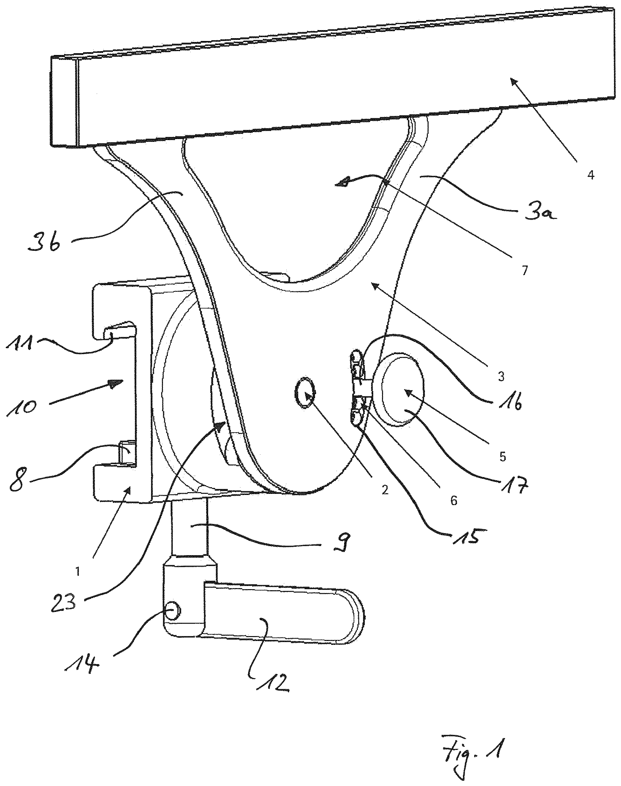

Die in den Figuren 1, 2 und 3 dargestellte Adaptiervorrichtung weist einen Spannkloben 1 als Klemmvorrichtung auf, mit dem die Adaptiervorrichtung an eine in den Figuren nicht dargestellte Seitenschiene eines Operationstischs geklemmt werden kann. Der Spannkloben 1 verfügt über eine Nut 10, über die er auf die Seitenschiene aufgeschoben werden kann. Die Nut 10 ist auf ihrer einen Seitenflanke mit einer Hinterschneidung ausgebildet, die durch einen eine in die Nut 10 eingebrachte Seitenschiene übergreifenden Vorsprung oder Leiste 11 gebildet ist. An der diesem gegenüberliegenden Seitenflanke der Nut 10 ist eine Druckplatte 8 angeordnet. Diese ist mittels einer Spannschraube 9 gegen eine in der Nut 10 eingebrachte Seitenschiene eines Operationstischs spannbar, so dass durch ein Anziehen der Spannschraube 9 die Druckplatte 8 gegen eine Seitenschiene gepresst wird, wodurch der Spannkloben 1 auf dieser fest verklemmt wird. Durch Lösen der Spannschraube 9 wird die Druckplatte 8 gegenüber der Seitenschiene gelockert, und der Spannkloben kann auf der Seitenschiene verschoben oder von dieser gelöst werden. Die Verwendung der Druckplatte 8 dient einer Verringerung der Flächenpressung auf die Seitenschiene und der Bildung von Druckstellen.

Um eine nutzerseitige Handhabung der Spannschraube 9 insbesondere ohne Werkzeug zu ermöglichen und zu erleichtern, ist diese mit einem Hebel 12 ausgestattet, über den die Spannschraube einfach und sicher angezogen und gelöst werden kann. Der Hebel 12 ist mittels eines Stiftes 14 an der Spannschraube 9 schwenkbar gelagert, so dass er, um diese anzuziehen oder zu lösen, quer zur Längsachse der Spannschraube 9 ausgerichtet und andernfalls in eine die Bedienung der Adaptiervorrichtung nicht behindernde Stellung, im Wesentlichen in Längsrichtung der Spannschraube 9 orientiert, gebracht werden kann. Anstelle der Spannschraube 9 kann auch ein Schnellspannelement genutzt werden, wie es aus dem Radsport zur Befestigung von Laufrädern in Radgabeln bekannt ist.

In dem Spannkloben 1 ist eine Schwenkachse 2 angeordnet, die auf der Ebene der mit der Nut 10 formschlüssig zusammenwirkenden Seitenschiene senkrecht steht, d. h. senkrecht zum Nutengrund. Die Schwenkachse ragt an der der Nut 10 abgewandten Seite des Spannklobens 1 über diesen hinaus und trägt einen Arm 3. Der Arm 3 ist auf der Schwenkachse 2 um diese herum drehbar aufgenommen, so dass zwischen dem Arm 3 und dem Spannkloben 1 ein Gelenk 24, hier in Form eines Drehgelenks/Scharniers, ausgebildet ist. Folglich sind der Spannkloben 1 und der Arm relativ zueinander um die Schwenkachse 2 schwenkbar.

Um ein Kugelgelenk auszubilden, kann das Ende der Schwenkachse 2 als Kugel ausgebildet sein, die in einer entsprechenden Kalotte des Arms 3 aufgenommen ist, so dass der Arm 3 nicht nur, wie in den Figuren dargestellt ist, um die Schwenkachse 2 mit einem Freiheitsgrad, sondern um die Kugel mit drei Freiheitsgraden schwenken kann.

Der Arm 3 weist zwei Armelemente oder Streben 3a und 3b auf. Diese sind winkelförmig zueinander angeordnet und bilden zwischen sich einen V-förmigen Freiraum 7, in den ein Nutzer bei einer Handhabung und insbesondere bei einem Schwenken und Justieren der Adaptiervorrichtung eingreifen kann und eines der Armelemente 3a, 3b oder beide als Handhabe verwenden kann. Zu diesem Zweck sind die zum Freiraum 7 gerichteten Kanten der Armelemente 3a, 3b abgerundet.

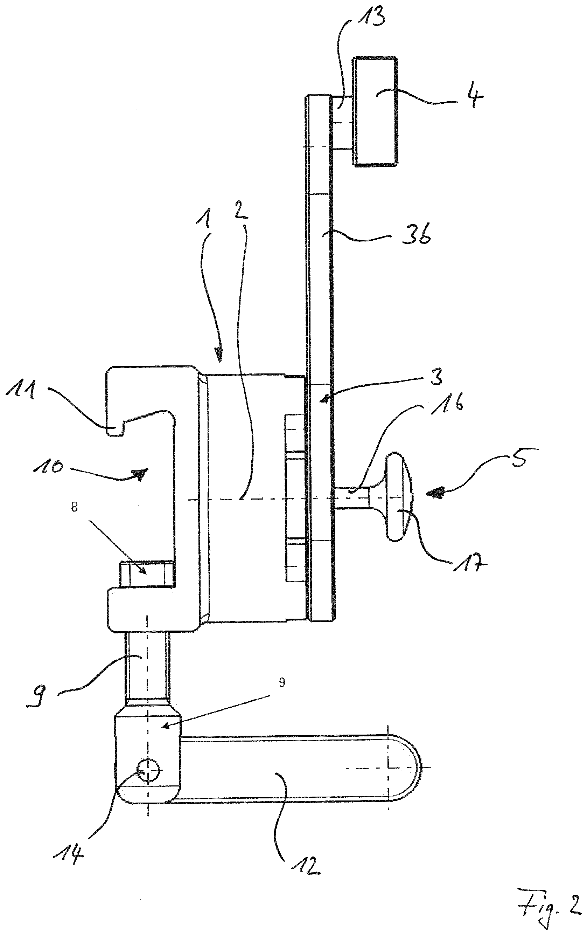

An ihren der Schwenkachse 2 abgewandten Enden tragen die beiden Armelemente 3a, 3b ein als eine Zusatzschiene dienendes Längsprofil 4. Dieses kann in beliebiger geeigneter Weise mit den Armelementen 3a, 3b verbunden oder aus einem Stück mit diesen ausgebildet sein. Fig. 2 zeigt, dass das Längsprofil 4 außerhalb der Ebene des Arms 3, an dessen vom Spannkloben 1 abgewandten Seite, angeordnet ist. Zwischen dem Längsprofil 4 und jedem Armelement 3a, 3b ist jeweils ein Distanzstück 13 angeordnet, das für einen ausreichenden Abstand des Längsprofils 4 von der Ebene des Arms 3 sorgt, so dass Zusatzeinrichtungen ohne Einschränkung durch die Armelemente 3a, 3b an dem Längsprofil 4 angeordnet und justiert und auf diesem verschoben werden können. Dadurch, dass der Arm 3 das Längsprofil 4 von der Schwenkachse 2 in orthogonaler Richtung mit Abstand trägt, kann die Höhe einer Operationstischauflage, also der Abstand von der Liegefläche für einen Patienten von der Seitenschiene des Operationstischs, ausgeglichen werden.

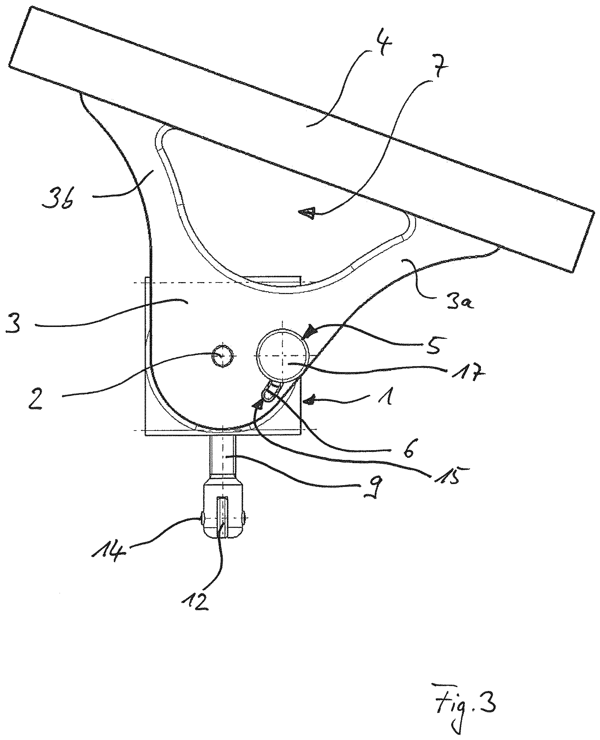

Die Länge des Längsprofils 4 ist mit LL bezeichnet. Diese Länge LL ist vorzugsweise etwas kürzer gehalten als die Länge LS eines in Figur 3 mit strichpunktierten Linien angedeuteten Seitenschienen-Segments25, an dem die Adaptiervorrichtung befestigt ist. Durch diese geometrische Anpassung ist dafür gesorgt, dass sich die einander benachbarten Schienen bei Relativ-Verschenkbewegungen nicht gegenseitig stören.

Bei den dargestellten Ausführungsbeispielen weist das Längsprofil 4 einen Querschnitt auf, der hinsichtlich Maßen und Geometrie dem genormten Querschnitt einer üblichen Seitenschiene eines Operationstischs entspricht. Auf diese Weise können übliche Zusatzeinrichtungen auf dem Längsprofil 4 angeordnet werden.

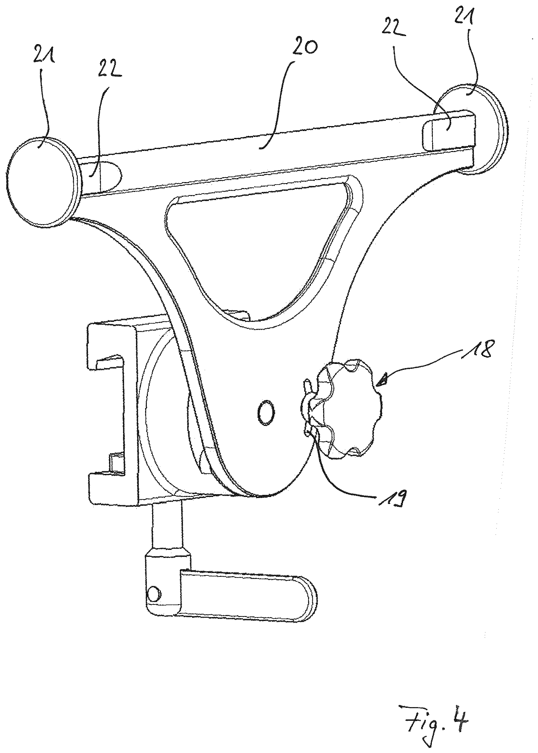

Ein dem Arm 3 zugewandte Gleitläche des Spannklobens 1 ist mit dem Bezugszeichen 26 (siehe Figur 2) versehen. Diese Gleitfläche 26 ist mit in den Figuren 1 und 4 erkennbaren Ausnehmungen 23, vorzugsweise in Form von Ausfräsungen, versehen, um eine Reinigung dieser Gleitfläche zu verbessern und zu erleichtern.

Die in den Figuren 1 bis 3 dargestellte Adaptiervorrichtung kann mittels eines Bolzens 5 in einer gewünschten Stellung arretiert werden. Die Arretierung erfolgt durch Formschluss des Bolzens mit im Arm (oder im Spannkolben 1) ausgebildeten Raststrukturen 6. In dem Arm 3 ist ein durchgehendes gebogenes Langloch 15 ausgebildet, dessen Krümmungsradius dem Abstand der Mittellinie des Langlochs 15 von der Mittelachse der Schwenkachse 2 entspricht und dessen Krümmungszentrum mit dieser Mittelachse zusammenfällt. Der Bolzen 5 ist mit einem in den Figuren nicht dargestellten Schaft fest mit dem Spannkloben 1 verbunden, beispielsweise in diesen eingeschraubt. Auf diesem Schaft befindet sich eine den Schaft umhüllende Hülse 16, die an ihrem vom Spannkloben 1 abgewandten Ende zu einem Betätigungsknopf 17 aufgeweitet ist. Das Außenmaß der Hülse 16 ist größer als das des Schaftes. Der Schaft und die Hülse 16 des Bolzens 5 sind mittels einer in den Figuren nicht dargestellten Feder gegeneinander derart vorgespannt, dass die Hülse 16 mit dem Betätigungsknopf 17 in Richtung des Spannklobens 1 gedrängt wird. Das Außenmaß der Hülse 16 ist derart auf das Innenmaß der Raststrukturen 6 abgestimmt, dass zwischen diesen Formschluss ist, wenn die Hülse 16 in eine der Raststrukturen 6 eingreift. Aufgrund dieses Formschlusses ist der Arm 3 gegenüber dem Spannkloben 1 arretiert. Die Federvorspannung drängt die Hülse 16 des Bolzens 5 stets in diesen Formschluss. Um die Arretierung zu lösen ergreift ein Nutzer den Betätigungsknopf 17 des Bolzens 5 und zieht diesen vom Spannkloben 1 gegen die Vorspannung der Feder fort. Die Hülse 16 löst sich dabei von den Raststrukturen 16, und wegen des kleineren Außenmaßes des Schafts kann der Arm 3 um die Schwenkachse 2 geschwenkt werden. Aufgrund der definierten Abstände der Raststrukturen 6 ist die Verstellbarkeit des Arms 3 gestuft.

Eine stufenlos verstellbare Ausgestaltung des Schwenkgelenks ist in Fig. 4 gezeigt. Bei dieser Ausführungsform ist anstelle des Bolzens 5 eine Klemmschraube 18 vorgesehen, die ein gebogenes Langloch 19 durchgreift und mit dem Spannkloben 1 direkt oder indirekt verschraubt ist. Das Langloch 19 ist nicht mit Raststrukturen versehen, sondern weist glatte Seitenwände auf und seine Krümmung und Position relativ zur Schwenkachse 2 entspricht der des Langlochs 15 der in den Figuren 1 bis 3 dargestellten Ausführungsform. Auf der dem Spannkloben 1 gegenüberliegenden Seite des Arms 3 ist die Klemmschraube 18 zu einer Schulter aufgeweitet, deren Durchmesser größer als das Innenmaß des Langlochs 19 ist. Durch ein Anziehen der Klemmschraube 18 wird der Arm 3 zwischen dem Spannkloben 1 und der Schulter der Klemmschraube 18 festgeklemmt und arretiert. Die Arretierung erfolgt durch Kraftschluss.

Ein weiterer Unterschied der in Fig. 4 dargestellten Ausführungsform gegenüber derjenigen der Figuren 1 bis 3 besteht darin, dass das Längsprofil 4 nicht mit den Querschnitt einer genormten Seitenschiene eines Operationstischs, sondern als Rundstange 20 mit Endanschlägen 21 auf beiden Seiten sowie mit Ausnehmungen 22 oder Abflachungen 22 ausgebildet ist. Die Endanschläge 21 verhindern, dass eine Zusatzeinrichtung versehentlich von der Rundstange 20 gleiten kann. Die Ausnehmungen 22 ermöglichen das Montieren und Demontieren von Zusatzeinrichtungen an bzw. von der Rundstange 20, wobei die Zusatzeinrichtungen nach dem Schlüssel-Schloss-Prinzip passend zu den Ausnehmungen 22 gestaltet sind und nur im Bereich dieser Ausnehmungen 22 montiert oder demontiert werden können. Selbstverständlich liegt es im Rahmen der Erfindung, dass die Rundstange 20 nach Fig. 4 mit der gestuften Arretierung nach den Figuren 1 bis 3 und die stufenlose Arretierung nach Fig. 4 mit dem Längsprofil 4 nach den Figuren 1 bis 3 kombiniert ist.

Bis auf die beiden vorgenannten Unterschiede entspricht die Adaptiervorrichtung der Fig. 4 im Übrigen der in den Figuren 1 bis 3 dargestellten Adaptiervorrichtung.

## Patentansprüche

1. Adaptiervorrichtung zur lösbaren Befestigung von Halteinstrumenten, Zusatzinstrumenten, Instrumentenzureichungstischen und dergleichen an einer Seitenschiene eines Operationstischs, mit
einer Klemmvorrichtung (1), mittels der die Adaptiervorrichtung an der Seitenschiene des Operationstischs klemmbar ist,
einem Längsprofil (4), auf dem Halteinstrumente, Zusatzinstrumente, Instrumentenzureichungstische und dergleichen lösbar zu befestigen sind,
**dadurch gekennzeichnet, dass** ein arretierbares Gelenk (24), welches mit wenigstens einem Schwenk-Freiheitsgrad (Drehachse 2) ausgestattet und zwischen der Klemmvorrichtung (1) und dem Längsprofil (4) angeordnet ist, so dass das Längsprofil (4) relativ zur Klemmvorrichtung (1) ausrichtbar ist.

2. Adaptiervorrichtung nach Anspruch 1, **dadurch gekennzeichnet, dass** das Gelenk (24) eine Verschwenkung des Längsprofils in Form einer Nickbewegung vorzugsweise in einer vertikalen Ebene erlaubt.

3. Adaptiervorrichtung nach Anspruch 1 oder 2, **dadurch gekennzeichnet, dass** das Gelenk (24) mittels Formschluss arretierbar ist, vorzugsweise indem ein Rastelement (5,16,17) in eine Rastausnehmung (6) oder in eine von mehreren Rastausnehmungen (6) des Gelenks (24) lösbar eingreift.

4. Adaptiervorrichtung nach Anspruch 3, **dadurch gekennzeichnet, dass** das Rastelement (5,16,17) ein federbelasteter Zugbolzen (16) ist, der in seiner Ruhelage, in die er vorgespannt ist, in die Rastausnehmung (6) oder in eine der Rastausnehmungen (6) eingreift und durch Zug gegen die Federbelastung von der Rastausnehmung (6) gelöst werden kann.

5. Adaptiervorrichtung nach Anspruch 1 oder 2, **dadurch gekennzeichnet, dass** das Gelenk (24) mittels Kraftschluss arretierbar ist, vorzugsweise indem eine Spannschraube (18) das Gelenk (24) verklemmt.

6. Adaptiervorrichtung nach einem der vorhergehenden Ansprüche, **dadurch gekennzeichnet, dass** das Gelenk (24) ein Drehgelenk oder ein Kugelgelenk ist.

7. Adaptiervorrichtung nach einem der vorhergehenden Ansprüche, **dadurch gekennzeichnet, dass** die Klemmvorrichtung (1) einen Teil des Gelenks (24) ausbildet.

8. Adaptiervorrichtung nach einem der vorhergehenden Ansprüche, **dadurch gekennzeichnet, dass** die Klemmvorrichtung (1) ein Spannkloben ist.

9. Adaptiervorrichtung nach einem der vorhergehenden Ansprüche, des weiteren aufweisend einen Arm (3,3a,3b), der zwischen dem Gelenk (24) und dem Längsprofil (4) angeordnet ist und das Längsprofil (4) von der Schwenkachse (2) des Gelenks (24) mit Abstand trägt.

10. Adaptiervorrichtung nach Anspruch 9, **dadurch gekennzeichnet, dass** der Arm (3,3a,3b) einen Freiraum (7) ausbildet, der bei einer Justierung oder Verstellung der Adaptiervorrichtung als Handgriff nutzbar ist.

11. Adaptiervorrichtung nach einem der vorhergehenden Ansprüche, **dadurch gekennzeichnet, dass** der Querschnitt des Längsprofils (4) dem Querschnitt einer genormten Seitenschiene eines Operationstischs entspricht oder rund ist.

12. Adaptiervorrichtung nach einem der vorhergehenden Ansprüche, **dadurch gekennzeichnet, dass** die Klemmvorrichtung (1) eine Druckplatte (8) aufweist, die mittels einer Spannschraube (9) gegenüber einer Seitenschiene des Operationstisches verspannbar ist.

13. Adaptiervorrichtung nach einem der vorhergehenden Ansprüche, **dadurch gekennzeichnet, dass** das Längsprofil (4) endseitige Anschläge (21) aufweist.

14. Adaptiervorrichtung nach einem der vorhergehenden Ansprüche, **dadurch gekennzeichnet, dass** das Längsprofil (4) Ausnehmungen (22) aufweist, beispielsweise in Form von Abflachungen oder Einschnitten, in die an dem Längsprofil (4) angeordnete Halteinstrumente, Zusatzinstrumente, Instrumentenzureichungstische und dergleichen nach dem Schlüssel-Schloss-Prinzip eingreifen.

15. Verwendung der Adaptiervorrichtung nach einem der Ansprüche 1 bis 14 für einen Operationstisch mit zumindest einer segmentierten Seitenschiene, **dadurch gekennzeichnet, dass** die Länge (LL) des Längsprofils (4) kürzer ist als die Länge (LS) des Seitenschienen-Segments, an dem die Adaptiervorrichtung befestigt ist.

## Claims

1. An adapter device for releasable mounting of holding instruments, auxiliary instruments, instrument feeding trays and the like on a side rail of an operating table, comprising
a clamping device (1) by means of which the adapter device can be clamped to the side rail of the operating table,
a longitudinal profile (4) on which holding instruments, auxiliary instruments, instrument feeding trays and the like are to be detachably fastened, **characterized in that**
a lockable joint (24) which is equipped with at least one pivoting degree of freedom (pivot axis 2) and is arranged between the clamping device (1) and the longitudinal profile (4) so that the longitudinal profile (4) can be aligned relative to the clamping device (1).

2. The adapter device according to claim 1, **characterized in that** the joint (24) allows for pivoting the longitudinal profile in the form of a pitch preferably in a vertical plane.

3. The adapter device according to claim 1 or 2, **characterized in that** the joint (24) can be locked by means of form closure, preferably **in that** a locking element (5, 16, 17) detachably engages in a locking recess (6) or in one of plural locking recesses (6) of the joint (24).

4. The adapter device according to claim 3, **characterized in that** the locking element (5, 16, 17) is a spring-loaded tension bolt (16) which, in its idle position into which it is biased, engages in the locking recesses (6) or in one of the locking recesses (6) and can be released from the locking recess (6) by pulling against the spring load.

5. The adapter device according to claim 1 or 2, **characterized in that** the joint (24) is lockable via force closure, preferably **in that** a tightening screw (18) clamps the joint (24).

6. The adapter device according to any one of the preceding claims, **characterized in that** the joint (24) is a pivot joint or a ball joint.

7. The adapter device according to any one of the preceding claims, **characterized in that** the clamping device (1) forms part of the joint (24).

8. The adapter device according to any one of the preceding claims, **characterized in that** the clamping device (1) is a vise.

9. The adapter device according to any one of the preceding claims, further comprising an arm (3, 3a, 3b) arranged between the joint (24) and the longitudinal profile (4) and supporting the longitudinal profile (4) at a distance from the pivot axis (2) of the joint (24).

10. The adapter device according to claim 9, **characterized in that** the arm (3, 3a, 3b) forms a free space (7) adapted to be used as a handle for adjusting or adapting the adapter device.

11. The adapter device according to any one of the preceding claims, **characterized in that** the cross-section of the longitudinal profile (4) corresponds to the cross-section of a standardized side rail of an operating table or is round.

12. The adapter device according to any one of the preceding claims, **characterized in that** the clamping device (1) includes a pressure plate (8) which is adapted to be braced by means of a tightening screw (9) vis-à-vis a side rail of the operating table.

13. The adapter device according to any one of the preceding claims, **characterized in that** the longitudinal profile (4) has end-side stops (21).

14. The adapter device according to any one of the preceding claims, **characterized in that** the longitudinal profile (4) includes recesses (22), for example in the form of flattened portions or indentations in which holding instruments, auxiliary instruments, instrument feeding trays and the like arranged on the longitudinal profile (4) engage in accordance with the lock-and-key principle.

15. Use of the adapter device according to any one of the claims 1 to 14 for an operating table comprising at least one segmented side rail, **characterized in that** the length (LL) of the longitudinal profile (4) is shorter than the length (LS) of the side rail segment to which the adapter device is fastened.

## Revendications

1. Dispositif adaptateur servant à fixer de manière détachable des instruments de maintien, des instruments supplémentaires, des tables permettant d'atteindre des instruments et similaires, au niveau d'un rail latéral d'une table d'opération, avec
un dispositif de serrage (1), au moyen duquel le dispositif adaptateur peut être serré au niveau du rail latéral de la table d'opération,
un profil longitudinal (4), sur lequel des instruments de maintien, des instruments supplémentaires, des tables permettant d'atteindre des instruments et similaires sont à fixer de manière détachable,
**caractérisé en ce que**
une articulation (24) pouvant être bloquée, qui est équipée d'au moins un degré de liberté de pivotement (axe de rotation 2) et qui est disposée entre le dispositif de serrage (1) et le profil longitudinal (4) de sorte que le profil longitudinal (4) puisse être orienté par rapport au dispositif de serrage (1).

2. Dispositif adaptateur selon la revendication 1, **caractérisé en ce que** l'articulation (24) permet un pivotement du profil longitudinal sous la forme d'un déplacement de tangage de préférence dans un plan vertical.

3. Dispositif adaptateur selon la revendication 1 ou 2, **caractérisé en ce que** l'articulation (24) peut être bloquée au moyen d'une complémentarité de forme, de préférence **en ce qu'**un élément d'enclenchement (5, 16, 17) vient en prise de manière détachable avec un évidement d'enclenchement (6) ou avec un parmi plusieurs évidements d'enclenchement (6) de l'articulation (24).

4. Dispositif adaptateur selon la revendication 3, **caractérisé en ce que** l'élément d'enclenchement (5, 16, 17) est un boulon tirant (16) soumis à la charge d'un ressort, qui vient en prise, dans sa position de repos, dans laquelle il est précontraint, avec l'évidement d'enclenchement (6) ou avec un des évidements d'enclenchement (6) et peut être détaché de l'évidement d'enclenchement (6) par traction à l'encontre de la charge exercée par le ressort.

5. Dispositif adaptateur selon la revendication 1 ou 2, **caractérisé en ce que** l'articulation (24) peut être bloquée au moyen d'une liaison à force, de préférence **en ce qu'**une vis de serrage (18) coince l'articulation (24).

6. Dispositif adaptateur selon l'une quelconque des revendications précédentes, **caractérisé en ce que** l'articulation (24) est une articulation tournante ou une articulation sphérique.

7. Dispositif adaptateur selon l'une quelconque des revendications précédentes, **caractérisé en ce que** le dispositif de serrage (1) réalise une partie de l'articulation (24).

8. Dispositif adaptateur selon l'une quelconque des revendications précédentes, **caractérisé en ce que** le dispositif de serrage (1) est un piston de serrage.

9. Dispositif adaptateur selon l'une quelconque des revendications précédentes, présentant par ailleurs un bras (3, 3a, 3b), qui est disposé entre l'articulation (24) et le profil longitudinal (4) et qui supporte le profil longitudinal (4) à distance de l'axe de pivotement (2) de l'articulation (24).

10. Dispositif adaptateur selon la revendication 9, **caractérisé en ce que** le bras (3, 3a, 3b) réalise un espace libre (7), qui peut être utilisé en tant que poignée lors d'un ajustement ou d'un réglage du dispositif adaptateur.

11. Dispositif adaptateur selon l'une quelconque des revendications précédentes, **caractérisé en ce que** la section transversale du profil longitudinal (4) correspond à la section transversale d'un rail latéral normé d'une table d'opération ou est ronde.

12. Dispositif adaptateur selon l'une quelconque des revendications précédentes, **caractérisé en ce que** le dispositif de serrage (1) présente une plaque de pression (8), qui peut être enserrée au moyen d'une vis de serrage (9) par rapport à un rail latéral de la table d'opération.

13. Dispositif adaptateur selon l'une quelconque des revendications précédentes, **caractérisé en ce que** le profil longitudinal (4) présente des butées (21) situées côté extrémité.

14. Dispositif adaptateur selon l'une quelconque des revendications précédentes, **caractérisé en ce que** le profil longitudinal (4) présente des évidements (22), par exemple sous la forme de parties plates ou d'incisions, avec lesquelles les instruments de maintien, instruments supplémentaires, tables permettant d'accéder aux instruments et similaires disposés au niveau du profil longitudinal (4) viennent en prise selon le principe clé-serrure.

15. Utilisation du dispositif adaptateur selon l'une quelconque des revendications 1 à 14 pour une table d'opération avec au moins un rail latéral segmenté, **caractérisée en ce que** la longueur (LL) du profil longitudinal (4) est plus courte que la longueur (LS) du segment de rail latéral, au niveau duquel le dispositif adaptateur est fixé.
